# EUROPEAN PATENT APPLICATION

(11) **EP 4 850 735 A2**
(43) Date of publication of application: **09.12.2026**
(21) Application number: 26207686.2
(22) Date of filing: 14.04.2022
(51) Int. Cl.: A61M 16/20

(54) **SYSTEMS FOR MEASURING PATIENT LUNG PRESSURE**

(30) Priority: 15.04.2021 US 202163175405 P
(62) Document number(s) of the earlier application(s) in accordance with Art. 76 EPC: 22789130.6 / 4 323 041
(71) Applicant: Ventec Life Systems, Inc., Bothell, WA 98021 (US)
(72) Inventor: CIPOLLONE, Joseph, Bothell, Washington 98021 (US); AHMAD, Samir Saleh, Bothell, Washington 98021 (US); HOLMES, Michael B., Bothell, Washington 98021 (US); SESMUNDO, Jason, Bothell, Washington 98021 (US)
(74) Representative: Mewburn Ellis LLP

(57) **Abstract**

The present technology is directed to systems and methods for measuring patient lung pressure in a patient during pressure-controlled or volume-controlled ventilation. For example, the present technology includes operating a ventilator blower at a first speed during an inspiratory phase of a breath to direct gas from the ventilator to the patient along a flow path, and, after the inspiratory phase and before an expiratory phase of the breath, operating the blower at a second speed less than the first speed to achieve a zero-flow state in the flow path during which gas neither flows into nor out of the patient's lungs. During the zero-flow state, the pressure in the flow path is equal or at least approximately equal to the patient lung pressure. Accordingly, pressure can be measured at any position along the flow path during the zero-flow state to determine patient lung pressure.

## Description

### CROSS-REFERENCE TO RELATED APPLICATION(S)

The present application claims priority to U.S. Provisional Application No. 63/175,405, filed April 15, 2021, and titled "SYSTEMS AND METHODS FOR MEASURING PATIENT LUNG PRESSURE IN VOLUME-CONTROLLED VENTILATION," the disclosure of which is incorporated by reference herein in its entirety.

### TECHNICAL FIELD

The present technology is generally directed to ventilator systems and methods of use, and in particular to measuring patient lung pressure and other physiologic metrics.

### BACKGROUND

Mechanical ventilators are typically connected to a patient using a patient circuit. Once connected to the patient, the ventilators drive inspiratory gases into the patient's lungs to assist with the patient's breathing. Gas flow can be controlled either using pressure-controlled ventilation or volume-controlled ventilation. In pressure-controlled ventilation, the ventilator delivers air to the patient until a predetermined pressure is reached. Once the predetermined pressure is reached, an expiratory valve in the patient circuit opens, reducing pressure in the patient circuit and enabling gases to flow out of the patient's lungs and exit the patient circuit via the expiratory valve. In volume-controlled ventilation, the ventilator delivers a predetermined volume of air to the patient. Once the predetermined volume of air is delivered to the patient, flow is reduced, and air naturally flows out of the patient's lungs back toward the ventilator.

### BRIEF DESCRIPTION OF THE DRAWINGS

Many aspects of the present technology can be better understood with reference to the following drawings. The components in the drawings are not necessarily to scale. Instead, emphasis is placed on clearly illustrating the principles of the present technology.
FIG. 1 is a schematic diagram of a ventilator system configured in accordance with embodiments of the present technology.
FIG. 2 illustrates a flow-waveform graph, a pressure-waveform graph, and a blower-speed graph for three respiratory cycles generated in accordance with embodiments of the present technology.
FIG. 3 is an enlarged view of a portion of the flow-waveform graph, pressure-waveform graph, and blower-speed graph of FIG. 2 illustrating a hold maneuver for measuring patient lung pressure in accordance with embodiments of the present technology.
FIG. 4A is a schematic diagram illustrating a ventilator control module for use with an active patient circuit and configured to perform a hold maneuver for measuring patient lung pressure in accordance with embodiments of the present technology.
FIG. 4B is a schematic diagram illustrating a ventilator control module for use with a passive patient circuit and configured to perform a hold maneuver for measuring patient lung pressure in accordance with embodiments of the present technology.
FIG. 5 is a schematic illustration of a ventilator configured to perform a hold maneuver for measuring patient lung pressure and configured in accordance with embodiments of the present technology.
FIG. 6 is a flowchart of a method for measuring patient lung pressure in a patient during ventilation in accordance with embodiments of the present technology.

### DETAILED DESCRIPTION

The present technology is generally directed to systems and methods for measuring patient lung pressure during pressure-controlled or volume-controlled mechanical ventilation. In some embodiments, the present technology further provides systems and methods for calculating patient static compliance and/or patient airway resistance based on the measured patient lung pressure. For example, the present technology includes operating a ventilator blower at a first speed during an inspiratory phase of a breath to direct gas from the ventilator to the patient along a flow path, and, after the inspiratory phase and before an expiratory phase of the breath, operating the blower at a second speed less than the first speed to achieve a zero-flow state in the flow path during which gas neither flows into nor out of the patient's lungs. During the zero-flow state, the pressure in the flow path is equal or at least approximately equal to the patient lung pressure. Accordingly, pressure can be measured at any position along the flow path during the zero-flow state to determine patient lung pressure. The measured patient lung pressure can then be used to automatically calculate patient static compliance and/or patient airway resistance. As described in detail herein, such measurements may assist in monitoring a patient's pulmonary health during ventilation, selecting an appropriate therapy level for the patient, or the like.

Patient lung pressure (e.g., the pressure in the patient's lungs at any given point during the respiratory cycle) varies throughout an inspiratory/expiratory respiratory cycle. For example, pressure in the patient's lungs generally increases during the inspiratory phase of a breath as air moves into and inflates the lungs. At the transition between the inspiratory phase and the expiratory phase, patient lung pressure is generally at or proximate its peak value (e.g., also known as plateau pressure). Patient lung pressure then decreases during the expiratory phase as air moves out of the lungs and the lungs deflate.

Patient lung pressure cannot be directly measured during a standard inspiratory-expiratory respiration cycle. This is because patient lung pressure generally is not equal to pressure at the patient connection (or any other position along the flow path between the ventilator and the patient's mouth) during ventilation due to the resistance of the patient's airway (e.g., trachea). For example, pressure at the patient's mouth generally will be greater than patient lung pressure at any given moment during the inspiratory phase (e.g., due to patient airway resistance), and pressure at the patient's mouth will generally be less than patient lung pressure at any given moment of the expiratory phase (e.g., also due to patient airway resistance). Accordingly, measuring pressure at the patient's mouth (or at another position along the flow path between the ventilator and the patient's mouth) during the inspiratory or expiratory phase does not provide an accurate estimate of patient lung pressure. Therefore, patient lung pressure cannot be measured simply by placing a pressure sensor on a patient connection at the patient's mouth.

In some situations, patient lung pressure can be assessed using a hold maneuver immediately following the inspiratory phase. During the hold maneuver, an expiratory valve in the patient circuit is held in a closed position following termination of the inspiratory phase. This causes a zero-flow state in the patient circuit, during which air neither flows into nor out of the patient's lungs. Because there is zero flow, the patient airway resistance does not affect the pressure in the patient's lungs. Accordingly, the pressure measured during the zero-flow state (known as the "plateau pressure") along the flow path between the ventilator and the patient is representative of the patient's peak lung pressure.

However, not all ventilator systems incorporate an expiratory valve in the patient circuit that can be selectively closed to achieve and maintain a zero-flow state. The present technology therefore provides systems and methods for measuring patient lung pressure, such as in in systems without an actively controlled expiratory valve. In particular, and as described in detail below, the present technology automatically controls operation of the blower to achieve a zero-flow state during a hold maneuver to permit a plateau pressure, and thus patient lung pressure, to be measured.

Assessing plateau pressure is useful in setting ventilation therapy parameters. A plateau pressure that is too high (e.g., greater than about 20 cmH₂O, greater than about 25 cmH₂O, greater than about 30 cmH₂O, or another patient-specific parameter) during or at the end of a hold maneuver may indicate the patient's lungs are over-inflated, which can cause barotrauma and lead to lung damage. A plateau lung pressure that is too low during or at the end of a hold maneuver may indicate the patient is not receiving sufficient air during respiration. Accordingly, ventilation therapy parameters can be selected to achieve a patient lung pressure at the end of the inspiratory phase (e.g., the plateau pressure) within a clinically acceptable range for a particular patient.

Patient static compliance (e.g., the distensibility/ability of the lung to stretch or expand in volume for a given pressure) and patient airway resistance (e.g., the resistance to air flow generated by the patient's anatomy) can also be useful in setting ventilation therapy parameters and/or monitoring the state of the patient. For example, a low patient lung compliance may indicate the patient has a "stiff" or fibrotic lung, whereas a high patient lung compliance may indicate the patient's lungs are overly pliable. However, as with patient lung pressure, patient static compliance and patient airway resistance cannot be directly measured during pressure-controlled or volume-controlled ventilation. However, as described in detail below, the present technology can utilize the measured patient lung pressure values to calculate patient compliance and patient airway resistance.

Further aspects and advantages of the devices, methods, and uses will become apparent from the ensuing description that is given by way of example only.

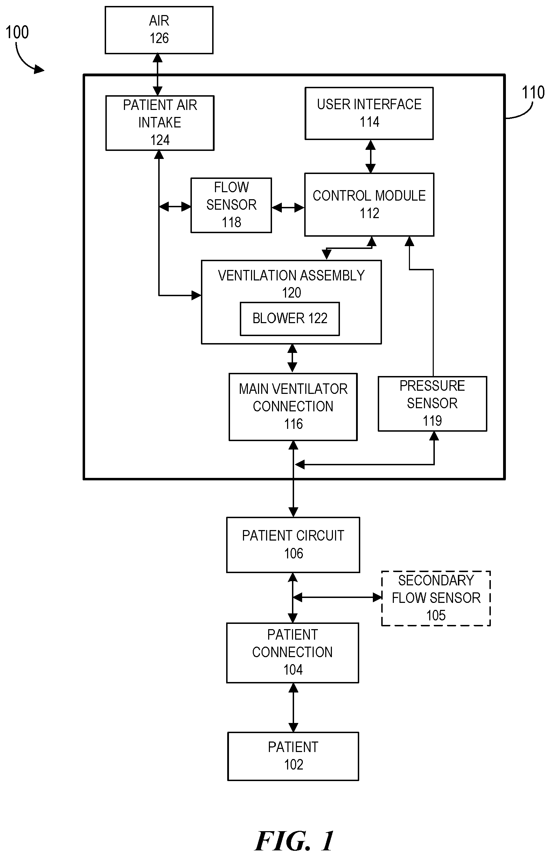

The terminology used in the description presented below is intended to be interpreted in its broadest reasonable manner, even though it is being used in conjunction with a detailed description of certain specific embodiments of the present technology. Certain terms may even be emphasized below; however, any terminology intended to be interpreted in any restricted manner will be overtly and specifically defined as such in this Detailed Description section. Additionally, the present technology can include other embodiments that are within the scope of the examples but are not described in detail with respect to FIGS. 1-6.

Reference throughout this specification to "one embodiment" or "an embodiment" means that a particular feature, structure, or characteristic described in connection with the embodiment is included in at least one embodiment of the present technology. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

Reference throughout this specification to relative terms such as, for example, "generally," "approximately," and "about" are used herein to mean the stated value plus or minus 10%. The term "substantially" or grammatical variations thereof refers to at least about 50%, for example, 75%, 85%, 95%, or 98%.

FIG. 1 is a schematic illustration of a ventilation system 100 ("the system 100") for providing ventilation therapy to a patient 102 and configured in accordance with embodiments of the present technology. The system 100 includes a ventilator 110, a patient circuit 106, and a patient connection 104. The ventilator 110 can be coupled to the patient 102 via the patient circuit 106 and the patient connection 104. For example, the patient circuit 106 can include a conduit or lumen (e.g., tubing) for transporting gases to and/or from the patient 102. The patient circuit 106 can include a passive patient circuit or an active patient circuit, such as those described in U.S. Patent Nos. 10,518,059 and 10,105,509, the disclosures of which are incorporated by reference herein in their entireties and for all purposes. The patient connection 104 can be any suitable interface coupled to the patient circuit 106 for delivering gases to the patient 102, such as a full rebreather mask, a partial rebreather mask, a nasal mask, a mouthpiece, a tracheal tube, or the like.

The ventilator 110 can include a ventilation assembly 120 having a blower 122 for providing inspiratory gases (e.g., "air 126") to the patient 102. The air 126 is received by the ventilator 110 via a patient air intake 124, which is coupled to the ventilation assembly 120. While identified as being "air," those of ordinary skill in the art will appreciate that the air 126 may include ambient air or pressurized air obtained from any source external to the ventilator 110. The air 126 may also optionally include concentrated oxygen, as described in U.S. Patent Nos. 10,245,406 and 10,315,002, the disclosures of which are incorporated by reference herein in their entireties and for all purposes.

The blower 122 controls the flow of air 126 to the patient 102. For example, during an inspiratory phase of a respiratory cycle, the blower 122 can direct air 126 to the patient 102 via a flow path that includes the patient air intake 124, the ventilation assembly 120, the main ventilator connection 116, the patient circuit 106, and the patient connection 104. Operation of the blower during the expiratory phase depends on the mode of therapy provided by the system 100. For example, in embodiments in which the system 100 is configured to provide positive end-expiratory pressure (PEEP or EPAP) therapy, the blower 122 also directs air 126 to the main ventilator connection 116 during the expiratory phase. This maintains a positive pressure within the patient's airways during the expiratory phase, which may reduce the risk that the patient's lungs will collapse during expiration. In some embodiments the ventilator 110 may receive expiratory gases during the expiratory phase. The ventilator 110 may purge expiratory gases via the patient air intake 124, and/or may have a separate outlet port (not shown) for venting patient expiratory gases.

The system 100 can further include one or more sensors, such as a flow sensor 118 and/or a pressure sensor 119. The flow sensor 118 can be positioned at any suitable position along the flow path between the patient air intake 124 and the patient connection 104. For example, in the illustrated embodiment the flow sensor 118 is positioned between the patient air intake 124 and the ventilation assembly 120. Regardless of its position, the flow sensor 118 is configured to measure the flow of gas (e.g., in liters per minute or LPM) between the patient air intake 124 and the patient 102. Likewise, the pressure sensor 119 can be positioned at any suitable position for measuring a pressure within the flow path between the patient air intake 124 and the patient 102. For example, the pressure sensor 119 can be positioned within the ventilator 110 between the ventilation assembly 120 and the main ventilator connection 116. In embodiments in which the patient circuit 106 is an active patient circuit, the system 100 can optionally include a secondary flow sensor 105 proximate the patient 202.

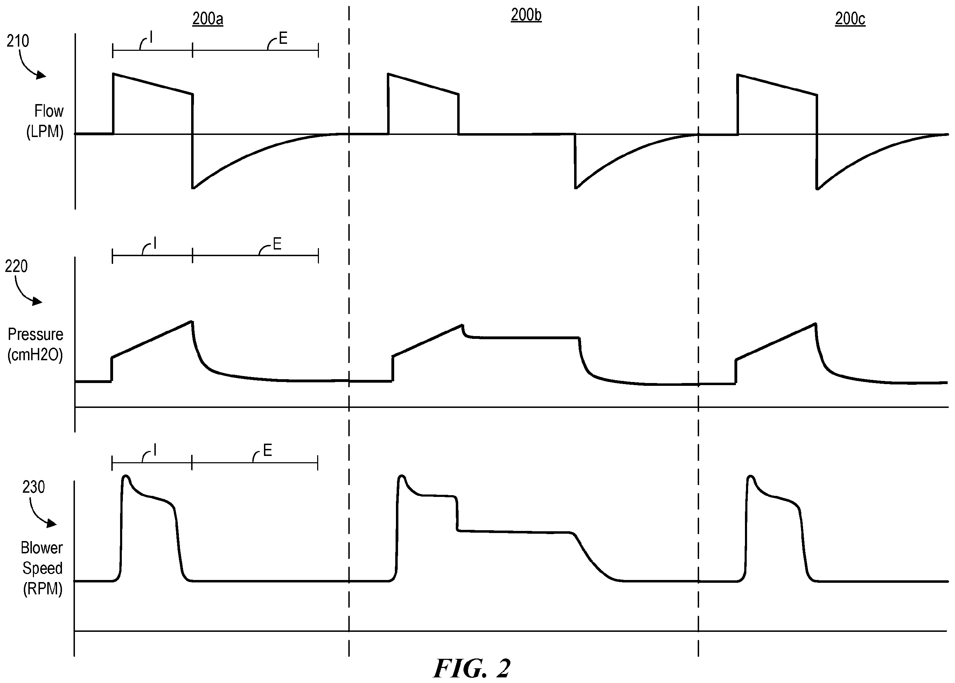

The ventilator 110 may further include a control module 112 for controlling operation of the ventilator 110. In particular, the control module 112 can generate one or more signals for controlling operation of the ventilation assembly 120, such as to automatically control a speed of the blower 122 (e.g., to provide a suitable flow of air 126 to the patient and/or to synchronize operation of the ventilator 110 with the patient's breath). For example, the control module 112 may direct the blower 122 to operate at a first speed during an inspiratory phase of a breath and a second speed slower than the first speed during an expiratory phase of the breath. The control module 112 may also receive signals from the flow sensor 118 and/or the pressure sensor 119. For example, in volume-controlled ventilation, the control module 112 may receive a signal from the flow sensor 118 during an inspiratory phase and, based on the received signal, automatically and in real-time calculate the volume of air delivered to the patient during the inspiratory phase. Once the calculated volume reaches a predetermined threshold, the control module 112 can control the ventilation assembly 120 to initiate the expiratory phase (e.g., by slowing the speed of the blower 122). Further yet, as described in detail with respect to FIGS. 2-6, the control module 112 can also automatically control the speed of the blower 122 based on the signals received from the flow sensor 118 to achieve a zero-flow state within the flow path during a hold maneuver in order to measure patient lung pressure.

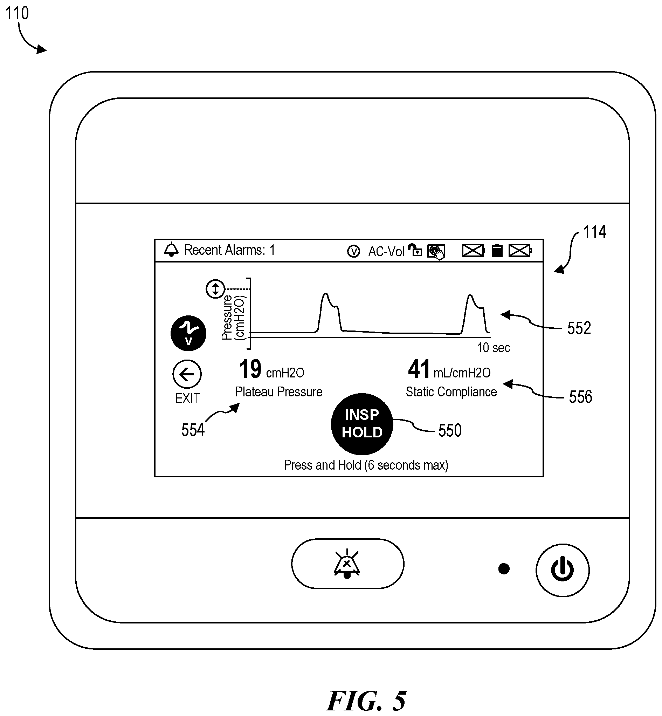

The ventilator 110 can further include a user interface 114. The user interface 114 is configured to receive input from a user (e.g., the patient, a caregiver, a clinician, or other user associated with the patient 102) and provide that input to the control module 112. The input received via the user interface 114 can include ventilator settings, parameters of operation, modes of operation, and the like. In a particular example, and as described in greater detail with respect to FIG. 5, a user may initiate a hold maneuver using the user interface 114 to measure patient lung pressure. The user interface 114 can further be configured to display information to the user and/or patient, including selected ventilator settings, parameters of operation, modes of operation, physiologic parameters, and the like. The user interface 114 can be any suitable user interface known in the art, such as a touch-screen having a digital display of ventilator settings and operating parameters.

The system 100 can optionally include additional features and functions beyond those described above. For example, the system 100 can include one or more of an oxygen assembly for providing supplemental oxygen to the patient 102, a cough-assist assembly for providing cough assistance to the patient 102, a nebulizer assembly for providing drug-therapy to the patient, a suction assembly for providing suction to the patient 102, or the like. In some embodiments, one or more of the foregoing assemblies (e.g., the cough-assist assembly) can be integrated into the ventilator 110 such that the system 100 can provide multiple respiratory therapies to the patient 102 without disconnecting the patient from the patient circuit 106. Additional features of ventilators suitable for use with the present technology are described in U.S. Patent No. 9,956,371, the disclosure of which is incorporated by reference herein in its entirety and for all purposes.

As described below with respect to FIGS. 2-6, the system 100 can be used to measure and/or calculate patient lung pressure, patient static compliance, and/or patient airway resistance during mechanical ventilation. In particular, patient lung pressure can be measured by controlling operation of the blower 122 to reduce net flow in the system 100 to zero following the inspiratory phase. Patient static compliance and patient airway resistance can be calculated using the measured patient lung pressure.

FIG. 2 includes a first graph 210 showing a representative flow waveform for three respiratory cycles 200a-c during volume-controlled ventilation (e.g., using the system 100 shown in FIG. 1), a second graph 220 showing a representative pressure waveform for the same three respiratory cycles 200a-c, and a third graph 230 showing the blower (e.g., the blower 122 shown in FIG. 1) speed for the same three respiratory cycles 200a-c. The first cycle 200a and the third cycle 200c are standard respiratory cycles having an inspiratory phase I immediately followed by an expiratory phase E. In the first cycle 200a and the third cycle 200c, flow transitions from a positive value during the inspiratory phase I to a negative value during the expiratory phase E, pressure rises during the inspiratory phase I and falls during the expiratory phase E, and the blower has a burst of speed during the inspiratory phase I and falls to a consistent level during the expiratory phase E to maintain positive end expiratory pressure.

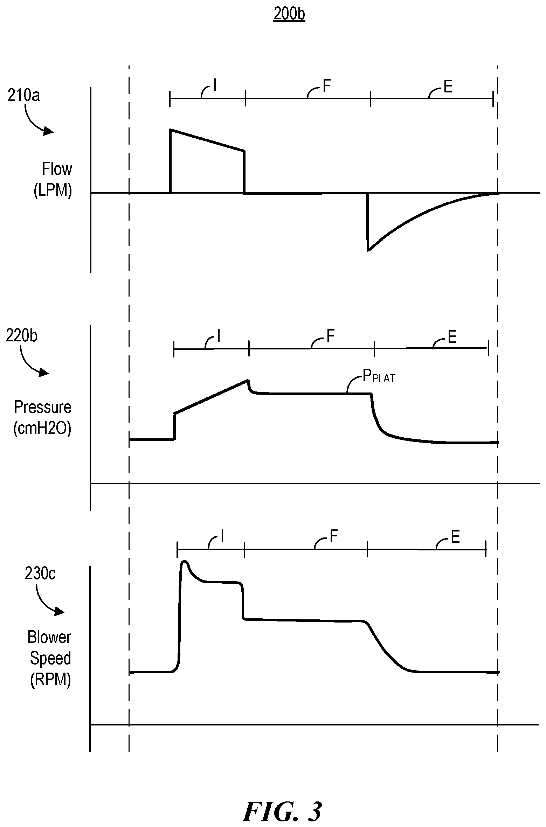

Unlike the first and third cycles 200a and 200c, the second cycle 200b includes a hold maneuver used to measure peak patient lung pressure (e.g., plateau pressure) in accordance with embodiments of the present technology. FIG. 3 is an enlarged view of the second cycle 200b showing the hold maneuver, with the other cycles omitted for clarity. Similar to the first and third cycles 200a and 200c, the second cycle 200b includes an inspiratory phase I and an expiratory phase E. However, the second cycle 200b also includes a zero-flow phase F between the inspiratory phase I and the expiratory phase E. During the zero-flow phase F, the blower is set (e.g., automatically set) to a speed that achieves and maintains zero flow in the flow path between the patient air intake 124 and the patient 102, as described in detail below. Because the flow is zero, the pressure plateaus during the zero-flow phase F (labeled as P_{PLAT} in FIG. 3), generally at a value less than the maximum pressure observed at the end of the inspiratory phase but greater than the baseline pressure observed at the end of the expiratory phase. Of note, the plateau pressure P_{PLAT} in the flow path during the zero-flow phase F is equal or at least approximately equal to the pressure in the patient's lungs. This is because with flow set to zero, the impact of any patient airway resistance and patient circuit resistance is null and therefore has no effect on pressure. Thus, pressure measured anywhere along the flow path (e.g., at the ventilation assembly 120, along the patient circuit 106, or at the patient connection 104) is equal to patient lung pressure. Accordingly, the plateau pressure P_{PLAT} measured during and/or at the end of the zero-flow phase F is equal or at least approximately equal to patient lung pressure, regardless of where the measurement is taken.

The third graph 230c shows operation of the blower 122 to maintain zero flow during the zero-flow phase F (e.g., gas neither flows into nor out of the patient's lungs). Of note, the blower 122 cannot simply turn off or even return to its baseline speed to achieve zero flow. If the blower 122 were turned off immediately following the inspiratory phase I, the patient's lungs would deflate and air would flow out of the patient's lungs and into the patient circuit, resulting in negative flow (e.g., flow toward the ventilation assembly 120). If the blower 122 were returned to its baseline speed immediately following the inspiratory phase I, such as in the first and third cycles 200a and 200c, the patient's lungs would still deflate in a normal expiratory event and air would flow out of the patient's lungs and into the patient circuit, also resulting in negative flow. If the blower 122 was maintained at or near its speed during the inspiratory phase I, the blower 122 would continue to move air into the patient's lungs, resulting in positive flow (e.g., flow toward the patient). Accordingly, to achieve zero flow, the blower 122 operates at an intermediate speed during the zero-flow phase F that is between its peak speed during the inspiratory phase I and its baseline speed during the expiratory phase E.

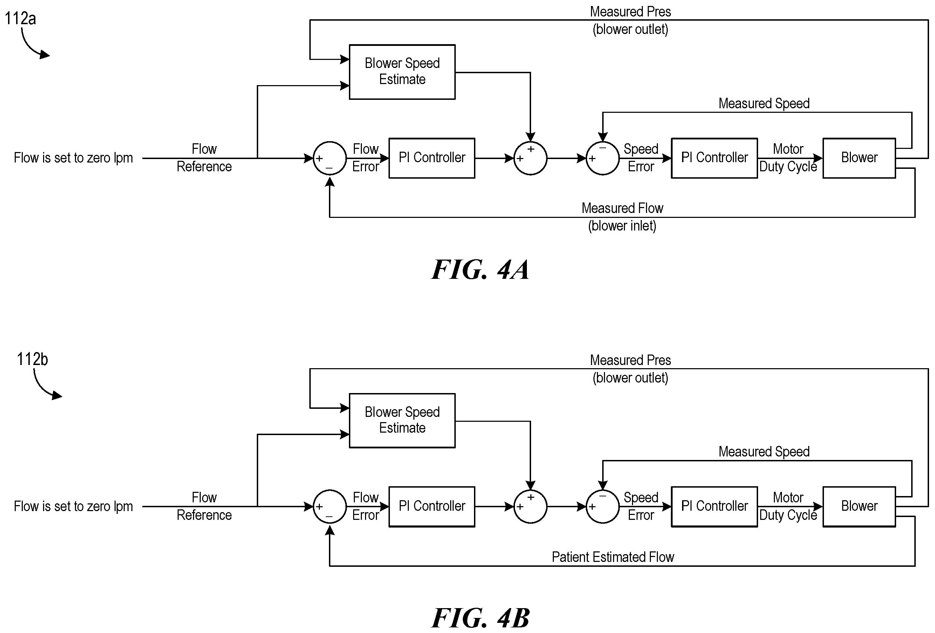

In particular, the system 100 can measure flow (e.g., via the flow sensor 118) and, based on the measured flow, automatically control the blower 122 to achieve and maintain the zero-flow state. For example, FIG. 4A is a flowchart illustrating a first ventilator control module 112a for use with an active patient circuit and configured to automatically control the speed of the blower to achieve a zero-flow state during the hold maneuver. For the hold maneuver, a flow reference value (e.g., a target flow) is set to zero. Based on the flow reference value and a measured pressure at the blower outlet, the control module 412a can estimate (e.g., using a look-up table having predetermined correlations between pressure, flow, and blower speed) a blower speed suitable for achieving the flow reference value. A first PI controller can also compare measured flow to the flow reference value to determine whether the flow is at the flow reference value. A second PI controller can automatically control the speed of the blower to the estimated speed (e.g., by controlling the duty cycle of the motor that drives the blower). Once the blower is operating at the estimated speed, the first PI controller can further fine-tune the speed of the blower to achieve and maintain the flow reference value.

FIG. 4B is a flowchart illustrating a second ventilator control module 112b for use with a passive patient circuit and configured to automatically control the speed of the blower to achieve a zero-flow state during the hold maneuver. The second ventilator control module 112b can be substantially similar to the first ventilator control module 112a described previously with respect to FIG. 4B. However, relative to the first ventilator control module 112a, the second ventilator control module 112b uses patient estimated flow to control speed of the blower to account for leaks in the passive patient circuit or any leaks at the patient connection.

As previously described, maintaining a zero-flow state in the system 100 following the inspiratory phase I causes the pressure within the flow path to equalize to the pressure within the patient's lung. Accordingly, the pressure measured during the zero-flow phase F along the flow path (e.g., the plateau pressure) is equal or at least approximately equal to the patient lung pressure.

Once patient lung pressure is measured, patient static compliance and patient airway resistance can be calculated using variations of the following equation, in which P is pressure, Q is flow, Rₚ is patient airway resistance, Vₜ is tidal volume, and C_{L} is patient lung compliance: $P = Q \times {R}_{p} + \frac{{V}_{t}}{{C}_{L}}$

During the hold maneuver, pressure P is patient lung pressure P_{L}, flow Q is zero, and patient airway resistance Rₚ is therefore null. Tidal volume Vₜ is known based on the volume of air delivered to the patient during the inspiratory phase. Accordingly, patient static compliance can be calculated using the following equation: ${C}_{L} = \frac{{V}_{t}}{{P}_{L}}$ Once patient static compliance is calculated, patient airway resistance can also be calculated.

In some embodiments, the system 100 can automatically determine patient lung pressure, patient static compliance, and/or patient airway resistance once a hold maneuver is initiated. For example, the control module 112 can include a computing module that automatically measures patient lung pressure during a hold maneuver and then automatically calculates patient static compliance and patient airway resistance based on the measured patient lung pressure. The system 100 can then display patient lung pressure, patient static compliance, and/or patient airway resistance (e.g., using the user interface 114).

FIG. 5 is a schematic illustration of the user interface 114 of the ventilator 110 of FIG. 1 illustrating a user-input hold maneuver control 550 (e.g., button, switch, toggle, etc.) for initiating a hold maneuver. In operation, a user can activate (e.g., press) the hold maneuver control 550 to initiate a hold maneuver that maintains a zero-flow state for measuring patient lung pressure. For example, when a user activates the hold maneuver control 550, the control module 112 (FIG. 1) can automatically control operation of the blower 122 to achieve a zero-flow state in the system 100, as described previously. In some embodiments, the hold maneuver continues for as long as the user activates (e.g., presses) the hold maneuver control 550, subject to a maximum hold period, described below.

In some embodiments, the user interface 114 can display a pressure-waveform graph 552 in real-time. Accordingly, when the user activates the hold maneuver control 550, the user can simultaneously view the pressure-waveform graph to ensure that a plateau pressure is achieved before releasing the hold maneuver control 550 and terminating the hold maneuver (hold maneuver pressure waveform not shown in FIG. 5). The user interface 114 can also display the most recently-measured plateau pressure metric 554 and the most recently-calculated static compliance metric 556.

A user can therefore initiate a hold maneuver using the user interface 114 to measure patient lung pressure at various times throughout the day (e.g., on-demand measurements). The duration of the hold maneuver can be preset (e.g., 1 second, 2 seconds, 3 seconds, 4 seconds, 5 seconds, or 6 seconds), or can be controlled by the user, as described above. For patient safety, the system 100 can include a maximum hold duration (e.g., 6 seconds) after which the system 100 returns to standard ventilation even if the user keeps the hold maneuver control 550 activated. In some embodiments, the system 100 can automatically initiate a hold maneuver at pre-set intervals. For example, the system 100 can be programmed to initiate a hold maneuver once per day, twice per day, three times per day, four times per day, five times per day, six times per day, etc.

Although the foregoing systems and techniques for measuring patient lung pressure and calculating patient static compliance and patient airway resistance are described with respect to the system 100, one skilled in the art will appreciate that the same techniques can be utilized with other ventilation systems that operate using a blower. Accordingly, the present technology is not limited to the system 100.

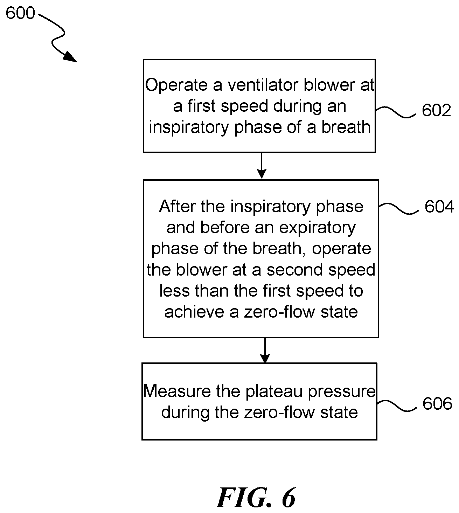

The present technology further includes methods for measuring patient lung pressure. For example, FIG. 6 is a flowchart of a method 600 for measuring patient lung pressure in a patient during pressure-controlled or volume-controlled ventilation. The method 600 can begin in step 602 by operating a ventilator blower (e.g., the blower 122 of the system 100) at a first speed during an inspiratory phase of the breath. The first speed can be sufficient to direct gas from the ventilator to the patient via a flow path that can include a patient circuit and a patient connection. During the inspiratory phase, the gas is supplied at a sufficient flow to inflate the patient's lungs.

After the inspiratory phase and before an expiratory phase of the breath, the method 600 can continue in step 604 by operating the blower at a second speed that is less than the first speed to achieve a zero-flow state in the flow path (e.g., preventing the patient from exhaling). This can include, for example, measuring the flow of gas in the flow path (e.g., using the flow sensor 118 of the system 100) and, based on the measured flow, automatically adjusting the speed of the blower to achieve and maintain the zero-flow state. The zero-flow state can be maintained for between about 1 second and about 6 seconds, such as for about 1 second, about 2 seconds, about 3 seconds, about 4 seconds, about 5 seconds, or about 6 seconds.

The method 600 further includes measuring the plateau pressure during the zero-flow state at step 606. This can include, for example, measuring pressure using a pressure sensor positioned within the ventilator. Of note, and as previously described, the plateau pressure measured during the zero-flow state is equal or at least approximately equal to the patient lung pressure. The measured plateau pressure can therefore be displayed to a user as the patient lung pressure. Patent static compliance can also be calculated using the measured patient lung pressure, as described above. Similarly, patient airway resistance can be calculated using the calculated static compliance, as also described above. After the plateau pressure is measured in step 606, the method 600 can continue by operating the blower at a third speed that is less than the second speed to permit the user to expire the gas delivered during the inspiratory phase of the breath.

### Examples

Several aspects of the present technology are set forth in the following examples:
1. A method for measuring patient lung pressure in a patient during ventilation using a ventilator connected to the patient by a patient circuit and a patient connection, the method comprising:
   operating a blower of the ventilator at a first speed during an inspiratory phase of a breath to direct gas from the ventilator to the patient along a flow path including the patient circuit and the patient connection;
   after the inspiratory phase and before an expiratory phase of the breath, operating the blower at a second speed less than the first speed to achieve a zero-flow state in the flow path during which gas neither flows into nor out of the lungs of the patient; and
   measuring a plateau pressure in the flow path during the zero-flow state, wherein the measured plateau pressure is equal or approximately equal to the patient lung pressure.
2. The method of example 1 wherein operating the blower at the second speed less than the first speed to achieve the zero-flow state comprises:
   measuring, via a flow sensor, gas flow in the flow path; and
   based on the measured gas flow, automatically adjusting the speed of the blower to achieve and maintain the zero-flow state.
3. The method of example 2 wherein the flow sensor is positioned within the ventilator.
4. The method of example 2 wherein the flow sensor is positioned within the patient circuit.
5. The method of any of examples 1-4 wherein measuring the plateau pressure in the flow path includes measuring the plateau pressure using a pressure sensor positioned within the ventilator.
6. The method of any of examples 1-5 wherein operating the blower at the second speed includes maintaining the zero-flow state for between about 1 second and about 6 seconds.
7. The method of any of examples 1-6, further comprising automatically calculating patient static compliance based on the measured plateau pressure.
8. The method of example 7, further comprising automatically calculating patient airway resistance based at least in part on the calculated patient static compliance.
9. The method of any of examples 1-8, further comprising operating the blower at a third speed after the zero-flow state is achieved and during the expiratory phase of the breath, wherein the third speed is less than the second speed.
10. A ventilator system, comprising:
   a ventilation assembly having a blower configured to control the flow of gas to a patient;
   a control module configured to control the blower, the control module including-one or more processors, and
      a memory storing instructions for performing a hold maneuver to measure patient lung pressure, wherein the instructions, when executed by the one or more processors, cause the ventilator system to perform operations comprising:
      operating the blower at a first speed during an inspiratory phase of a breath to direct gas from the ventilator to the patient along a flow path including a patient circuit and a patient connection, and
      after the inspiratory phase and before an expiratory phase of the breath, reducing the speed of the blower to achieve a zero-flow state in the flow path during which gas neither flows into nor out of the lungs of the patient; and
   a pressure sensor configured to measure a plateau pressure in the flow path during the zero-flow state, wherein the measured plateau pressure is equal or approximately equal to the patient lung pressure.
11. The system of example 10, further comprising a flow sensor configured to measure gas flow in the flow path, and wherein the operation of reducing the speed of the blower to achieve a zero-flow state further includes:
   receiving a signal from the flow sensor indicative of gas flow in the flow path, and
   based on the received signal, automatically adjusting the speed of the blower to achieve and maintain the zero-flow state.
12. The system of example 10 or 11 wherein the zero-flow state is maintained for between about 1 second and about 6 seconds.
13. The system of any of examples 10-12 wherein the operations further comprise automatically calculating patient static compliance based on the measured plateau pressure.
14. The system of example 13 wherein the operations further comprise automatically calculating patient airway resistance based at least in part on the calculated patient static compliance.
15. The system of any of examples 10-14 wherein the operations further comprise further reducing the speed of the blower after the zero-flow state is achieved and during the expiratory phase to permit patient expiration.
16. The system of any of examples 10-15, further comprising a user display configured to display the measured plateau pressure during the zero-flow state.
17. The system of any of examples 10-16, further comprising a user input for selectively initiating operation of the hold maneuver.

### Conclusion

The systems and methods described herein can be implemented with and/or distributed across computing architecture. For example, many of the systems described herein include a memory storing data, software modules, instructions, or the like. The memories described herein can include one or more of various hardware devices for volatile and non-volatile storage, and can include both read-only and writable memory. For example, a memory can comprise random access memory (RAM), various caches, CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory. In some embodiments, the memory is a non-transitory computer-readable storage medium that stores, for example, programs, software, data, or the like.

As one of skill in the art will appreciate from the disclosure herein, various components of the systems described above can be omitted without deviating from the scope of the present technology. Likewise, additional components not explicitly described above may be added to the systems without deviating from the scope of the present technology. For example, it will be appreciated that specific embodiments of the technology have been described herein for purposes of illustration, but well-known structures and functions have not been shown or described in detail to avoid unnecessarily obscuring the description of the embodiments of the technology. Moreover, although specific embodiments of, and examples for, the technology are described above for illustrative purposes, various equivalent modifications are possible within the scope of the technology as those skilled in the relevant art will recognize. For example, although steps are presented in a given order, alternative embodiments may perform steps in a different order. The various embodiments described herein may also be combined to provide further embodiments. Accordingly, the present technology is not limited to the configurations expressly identified herein, but rather encompasses variations and alterations of the described systems and methods.

Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Unless the context clearly requires otherwise, throughout the description and the examples, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof, means any connection or coupling, either direct or indirect, between two or more elements; the coupling of connection between the elements can be physical, logical, or a combination thereof. Additionally, the words "herein," "above," "below," and words of similar import, when used in this application, shall refer to this application as a whole and not to any particular portions of this application. Where the context permits, words in the above Detailed Description using the singular or plural number may also include the plural or singular number respectively. As used herein, the phrase "and/or" as in "A and/or B" refers to A alone, B alone, and A and B. Further, where specific integers are mentioned herein which have known equivalents in the art to which the embodiments relate, such known equivalents are deemed to be incorporated herein as if individually set forth.
The following clauses, which form part of the description, provide general expressions of the disclosure herein:
1. A method for measuring patient lung pressure in a patient during ventilation using a ventilator connected to the patient by a patient circuit and a patient connection, the method comprising:
   operating a blower of the ventilator at a first speed during an inspiratory phase of a breath to direct gas from the ventilator to the patient along a flow path including the patient circuit and the patient connection;
   after the inspiratory phase and before an expiratory phase of the breath, operating the blower at a second speed less than the first speed to achieve a zero-flow state in the flow path during which gas neither flows into nor out of the lungs of the patient; and
   measuring a plateau pressure in the flow path during the zero-flow state, wherein the measured plateau pressure is equal or approximately equal to the patient lung pressure.
2. The method of clause 1 wherein operating the blower at the second speed less than the first speed to achieve the zero-flow state comprises:
   measuring, via a flow sensor, gas flow in the flow path; and
   based on the measured gas flow, automatically adjusting the speed of the blower to achieve and maintain the zero-flow state.
3. The method of clause 2 wherein the flow sensor is positioned within the ventilator.
4. The method of clause 2 wherein the flow sensor is positioned within the patient circuit.
5. The method of clause 1 wherein measuring the plateau pressure in the flow path includes measuring the plateau pressure using a pressure sensor positioned within the ventilator.
6. The method of clause 1 wherein operating the blower at the second speed includes maintaining the zero-flow state for between about 1 second and about 6 seconds.
7. The method of clause 1, further comprising automatically calculating patient static compliance based on the measured plateau pressure.
8. The method of clause 7, further comprising automatically calculating patient airway resistance based at least in part on the calculated patient static compliance.
9. The method of clause 1, further comprising operating the blower at a third speed after the zero-flow state is achieved and during the expiratory phase of the breath, wherein the third speed is less than the second speed.
10. A ventilator system, comprising:
   a ventilation assembly having a blower configured to control the flow of gas to a patient;
   a control module configured to control the blower, the control module including-one or more processors, and
      a memory storing instructions for performing a hold maneuver to measure patient lung pressure, wherein the instructions, when executed by the one or more processors, cause the ventilator system to perform operations comprising:
      operating the blower at a first speed during an inspiratory phase of a breath to direct gas from the ventilator to the patient along a flow path including a patient circuit and a patient connection, and
      after the inspiratory phase and before an expiratory phase of the breath, reducing the speed of the blower to achieve a zero-flow state in the flow path during which gas neither flows into nor out of the lungs of the patient; and
   a pressure sensor configured to measure a plateau pressure in the flow path during the zero-flow state, wherein the measured plateau pressure is equal or approximately equal to the patient lung pressure.
11. The system of clause 10, further comprising a flow sensor configured to measure gas flow in the flow path, and wherein the operation of reducing the speed of the blower to achieve a zero-flow state further includes:
   receiving a signal from the flow sensor indicative of gas flow in the flow path, and
   based on the received signal, automatically adjusting the speed of the blower to achieve and maintain the zero-flow state.
12. The system of clause 10 wherein the zero-flow state is maintained for between about 1 second and about 6 seconds.
13. The system of clause 10 wherein the operations further comprise automatically calculating patient static compliance based on the measured plateau pressure.
14. The system of clause 13 wherein the operations further comprise automatically calculating patient airway resistance based at least in part on the calculated patient static compliance.
15. The system of clause 10 wherein the operations further comprise further reducing the speed of the blower after the zero-flow state is achieved and during the expiratory phase to permit patient expiration.
16. The system of clause 10, further comprising a user display configured to display the measured plateau pressure during the zero-flow state.
17. The system of clause 10, further comprising a user input for selectively initiating operation of the hold maneuver.

## Claims

1. A ventilator system, comprising:
a ventilation assembly having a blower configured to control the flow of gas to a patient along a flow path including a patient circuit and a patient connection during an inspiratory phase and an expiratory phase of a breath;
a control module configured to control the blower, the control module including-one or more processors, and
a memory storing instructions for performing a hold maneuver to measure patient lung pressure, wherein the instructions, when executed by the one or more processors, cause the ventilator system to perform operations comprising:
after the inspiratory phase and before the expiratory phase of the breath, adjusting the blower speed to achieve and maintain a zero-flow state in the flow path during which gas neither flows into nor out of the lungs of the patient;
a pressure sensor configured to measure a plateau pressure in the flow path during the zero-flow state, wherein the measured plateau pressure is equal or approximately equal to the patient lung pressure; and
a flow sensor configured to measure gas flow in the flow path,
wherein the operation of adjusting the blower speed to achieve and maintain the zero-flow state further includes:
receiving a signal from the flow sensor indicative of gas flow in the flow path, and
based on the received signal, (a) reducing the blower speed to achieve the zero-flow state, and (b) automatically adjusting the blower speed to maintain the zero-flow state.

2. The ventilator system of claim 1, wherein the instructions further cause the ventilator system to perform: operating the blower during the inspiratory phase of the breath to direct gas from the ventilator assembly to the patient along the flow path including the patient circuit and the patient connection.

3. The ventilator system of claim 1 or 2 wherein the operations further comprise automatically calculating patient static compliance based on the measured plateau pressure.

4. The ventilator system of claim 3 wherein the operations further comprise automatically calculating patient airway resistance based at least in part on the calculated patient static compliance.

5. The ventilator system of claim 1 wherein before adjusting the blower speed to achieve the zero-flow state, the control module is configured to control the blower during the inspiratory phase of the breath to direct gas from the ventilation assembly to the patient along the flow path.

6. The system of any of claims 1 to 5 wherein the operations further comprise, after maintaining the zero-flow state, further reducing the blower speed during the expiratory phase to permit patient expiration.

7. The system of any of claims 1 to 6, further comprising a user display configured to display the measured plateau pressure during the zero-flow state.

8. The ventilator system of claim 1, further comprising a user input, wherein the control module is configured to execute the instructions for measuring patient lung pressure in response to a user engaging the user input.

9. The ventilator system of claim 1 wherein the flow sensor is positioned within the ventilation assembly.

10. The ventilator system of claim 1 wherein the flow sensor is positioned within the patient circuit.

11. The ventilator system of claim 1 wherein the operations further include: estimating the blower speed needed to achieve the zero-flow state based on a flow reference value and a measured pressure at an outlet of the blower, wherein the flow reference value is zero; and reducing the blower speed to the estimated speed.

12. The ventilator system of claim 11 wherein estimating the blower speed based on the flow reference value and the measured pressure includes using a look-up table having predetermined correlations between pressure, flow, and blower speed.

13. The ventilator system of claim 1 wherein automatically adjusting the blower speed to maintain the zero-flow state is based at least in part on patient estimated flow.

14. The ventilator system of claim 1 wherein the zero-flow state is maintained for between about 1 second and about 6 seconds.
